# EUROPEAN PATENT APPLICATION

(11) **EP 4 206 225 A1**
(43) Date of publication of application: **05.07.2023**
(21) Application number: 21861597.9
(22) Date of filing: 25.08.2021
(51) Int. Cl.: C07K 16/20, A61K 39/395, A61P 33/06, C07K 16/46, G01N 33/53, G01N 33/569, C12N 15/13

(54) **ANTI-MALARIA PARASITE ANTIBODY**

(30) Priority: 26.08.2020 JP 2020142450; 04.03.2021 JP 2021034402
(71) Applicant: National University Corporation Ehime University, Matsuyama-shi, Ehime 790-8577 (JP); Sumitomo Pharma Co., Ltd., Osaka 541-0045 (JP)
(72) Inventor: TSUBOI, Takafumi, Matsuyama-shi, Ehime 790-8577 (JP); TAKASHIMA, Eizo, Matsuyama-shi, Ehime 790-8577 (JP); NAGAOKA, Hikaru, Matsuyama-shi, Ehime 790-8577 (JP); FUKUSHIMA, Akihisa, Osaka-shi, Osaka 554-0022 (JP)
(74) Representative: Mewburn Ellis LLP
(86) International application number: PCT/JP2021/031103
(87) International publication number: WO 2022/045173

(57) **Abstract**

The present disclosure includes a monoclonal antibody or antibody fragment that binds to an epitope consisting of 5 to 20 consecutive amino acids in the amino acid sequence of SEQ ID NO: 1, and a method of detecting or quantifying an Ripr-derived malaria vaccine antigen comprising contacting a sample with the monoclonal antibody or antibody fragment.

## Description

### TECHNICAL FIELD

The present application claims priority to Japanese Patent Application Nos. 2020-142450 and 2021-034402, the whole of which is herein incorporated by reference.

The present disclosure includes a monoclonal antibody or antibody fragment that binds to a malaria parasite-derived antigen.

### BACKGROUND ART

Malaria, which is an infection of a parasitic protozoa of Plasmodium such as Plasmodium falciparum, is widespread in tropical and subtropical regions. Malaria disease develops when malaria parasites enter into human bodies by using Anopheles as vectors and proliferate through the sporozoite stage, liver stage, and red blood cell stage. In each stage, malaria parasites produce proteins in the human body. Rh5 interacting protein (PlasmoDB gene code: PF3D7_0323400, http://plasmodb.org/), also called as Ripr, is one of proteins considered to be expressed in Plasmodium falciparum at the merozoite stage (Non-Patent documents 1 and 2). Ripr is suggested to be an antigen for malaria vaccines (Non-Patent documents 3 and 4 and Patent documents 1 to 5).

### PRIOR ART DOCUMENTS

### PATENT DOCUMENTS

Patent Document 1 US Patent Application Publication No. 2015/0366958
Patent Document 2: WO2012/061882
Patent Document 3: WO2015/144874
Patent Document 4: WO2014/174054
Patent Document 5: WO2018/088507

### NON-PATENT DOCUMENTS

Non-Patent Document 1: Nature (London) (1999), 400(6744), 532-538
Non-Patent Document 2: Nature (London, United Kingdom) (2002), 419(6906), 527-531
Non-Patent Document 3: Vaccine (2016), 34(46):5612-5622
Non-Patent Document 4: Scientific Reports (2020), 10(1):6573

### SUMMARY OF THE INVENTION

### PROBLEMS TO BE SOLVED BY THE INVENTION

Antibodies against malaria parasite proteins such as Ripr are expected to be able to attack malaria parasites in the body after malaria infection, and to treat malaria disease or prevent the development of malaria disease when malaria parasites invade the human body. An object of the present disclosure is to provide a monoclonal antibody or antibody fragment that is effective in treating or preventing malaria disease. A further object of the present disclosure is to provide a method of detecting or quantifying a malaria vaccine antigen for quality control during production of a malaria vaccine.

### SOLUTIONS TO THE PROBLEMS

Through intensive studies, the present inventors obtained a plurality of monoclonal antibodies using Ripr as an antigen, which is a protein derived from malaria parasites, and found some antibodies exhibiting an inhibitory activity on malaria parasite growth from these monoclonal antibodies, and completed the present invention.

The present disclosure provides, for example, the followings.
1. A monoclonal antibody or antibody fragment that binds to an epitope consisting of 5 to 20 consecutive amino acids in the amino acid sequence of SEQ ID NO: 1.
2. The monoclonal antibody or antibody fragment of item 1, wherein the epitope consists of a sequence contained in the amino acid sequence of SEQ ID NO: 2.
3. The monoclonal antibody or antibody fragment of item 1 or 2, comprising
   a heavy chain variable region comprising
      CDR1 consisting of the amino acid sequence of SEQ ID NO: 3 or an amino acid sequence in which one or two amino acid residues are independently substituted, deleted, added, or inserted in the amino acid sequence of SEQ ID NO: 3;
      CDR2 consisting of the amino acid sequence of SEQ ID NO: 4 or SEQ ID NO: 9 or an amino acid sequence in which one or two amino acid residues are independently substituted, deleted, added, or inserted in the amino acid sequence of SEQ ID NO: 4 or SEQ ID NO: 9; and
      CDR3 consisting of the amino acid sequence of SEQ ID NO: 5 or SEQ ID NO: 10 or an amino acid sequence in which one or two amino acid residues are independently substituted, deleted, added, or inserted in the amino acid sequence of SEQ ID NO: 5 or SEQ ID NO: 10; and
   a light chain variable region comprising
      CDR1 consisting of the amino acid sequence of SEQ ID NO: 6 or SEQ ID NO: 11 or an amino acid sequence in which one or two amino acid residues are independently substituted, deleted, added, or inserted in the amino acid sequence of SEQ ID NO: 6 or SEQ ID NO: 11;
      CDR2 consisting of the amino acid sequence of SEQ ID NO: 7 or an amino acid sequence in which one or two amino acid residues are independently substituted, deleted, added, or inserted in the amino acid sequence of SEQ ID NO: 7; and
      CDR3 consisting of the amino acid sequence of SEQ ID NO: 8 or SEQ ID NO: 12 or an amino acid sequence in which one or two amino acid residues are independently substituted, deleted, added, or inserted in the amino acid sequence of SEQ ID NO: 8 or SEQ ID NO: 12.
4. The monoclonal antibody or antibody fragment of any one of items 1-3, comprising
   a heavy chain variable region comprising
      CDR1 consisting of the amino acid sequence of SEQ ID NO: 3;
      CDR2 consisting of the amino acid sequence of SEQ ID NO: 4 or SEQ ID NO: 9; and
      CDR3 consisting of the amino acid sequence of SEQ ID NO: 5 or SEQ ID NO: 10; and
   a light chain variable region comprising
      CDR1 consisting of the amino acid sequence of SEQ ID NO: 6 or SEQ ID NO: 11;
      CDR2 consisting of the amino acid sequence of SEQ ID NO: 7; and
      CDR3 consisting of the amino acid sequence of SEQ ID NO: 8 or SEQ ID NO: 12.
5. The monoclonal antibody or antibody fragment of any one of items 1-4, comprising
   a heavy chain variable region comprising CDR1 consisting of the amino acid sequence of SEQ ID NO: 3, CDR2 consisting of the amino acid sequence of SEQ ID NO: 4, and CDR3 consisting of the amino acid sequence of SEQ ID NO: 5; and
   a light chain variable region comprising CDR1 consisting of the amino acid sequence of SEQ ID NO: 6, CDR2 consisting of the amino acid sequence of SEQ ID NO: 7, and CDR3 consisting of the amino acid sequence of SEQ ID NO: 8.
6. The monoclonal antibody or antibody fragment of any one of items 1-4, comprising
   a heavy chain variable region comprising CDR1 consisting of the amino acid sequence of SEQ ID NO: 3, CDR2 consisting of the amino acid sequence of SEQ ID NO: 9, and CDR3 consisting of the amino acid sequence of SEQ ID NO: 10; and
   a light chain variable region comprising CDR1 consisting of the amino acid sequence of SEQ ID NO: 11, CDR2 consisting of the amino acid sequence of SEQ ID NO: 7, and CDR3 consisting of the amino acid sequence of SEQ ID NO: 12.
7. The monoclonal antibody or antibody fragment of any one of items 1-6, comprising a heavy chain variable region comprising an amino acid sequence having at least 90% sequence identity with SEQ ID NO: 13 and a light chain variable region comprising an amino acid sequence having at least 90% sequence identity with SEQ ID NO: 14.
8. The monoclonal antibody or antibody fragment of item 7, comprising a heavy chain variable region comprising the amino acid sequence of SEQ ID NO: 13 and a light chain variable region comprising the amino acid sequence of SEQ ID NO: 14.
9. The monoclonal antibody or antibody fragment of any one of items 1-6, comprising a heavy chain variable region comprising an amino acid sequence having at least 90% sequence identity with SEQ ID NO: 15 and a light chain variable region comprising an amino acid sequence having at least 90% sequence identity with SEQ ID NO: 16.
10. The monoclonal antibody or antibody fragment of item 9, comprising a heavy chain variable region comprising the amino acid sequence of SEQ ID NO: 15 and a light chain variable region comprising the amino acid sequence of SEQ ID NO: 16.
11. The monoclonal antibody or antibody fragment of any one of items 1-10, comprising
   a heavy chain variable region comprising CDR1, CDR2 and CDR3 from the amino acid sequence of SEQ ID NO: 13 and a light chain variable region comprising CDR1, CDR2 and CDR3 from the amino acid sequence of SEQ ID NO: 14; or
   a heavy chain variable region comprising CDR1, CDR2 and CDR3 from the amino acid sequence of SEQ ID NO: 15 and a light chain variable region comprising CDR1, CDR2 and CDR3 from the amino acid sequence of SEQ ID NO: 16.
12. A monoclonal antibody or antibody fragment that competes with the monoclonal antibody or antibody fragment of any one of items 1 to 11 for binding to a polypeptide consisting of the amino acid sequence of SEQ ID NO: 1.
13. The monoclonal antibody or antibody fragment of any one of items 1 to 12, which is a chimeric antibody or fragment thereof.
14. The monoclonal antibody or antibody fragment of any one of items 1 to 12, which is a humanized antibody or fragment thereof.
15. The monoclonal antibody or antibody fragment of any one of items 1-14, which has a modified constant region.
16. The monoclonal antibody or antibody fragment of any one of items 1-15, which is conjugated to a drug.
17. A pharmaceutical composition for treating or preventing malaria disease, comprising the monoclonal antibody or antibody fragment of any one of items 1 to 16.
18. The monoclonal antibody or antibody fragment of any one of items 1 to 16 for use in treating or preventing malaria disease.
19. A method for treating or preventing malaria disease, comprising administering the monoclonal antibody or antibody fragment of any one of items 1 to 16 to a subject.
20. Use of the monoclonal antibody or antibody fragment of any one of items 1 to 16 for the manufacture of a medicament for treating or preventing malaria disease.
21. Use of the monoclonal antibody or antibody fragment of any one of items 1 to 16 for treating or preventing malaria disease.
22. A method of detecting or quantifying an Ripr-derived malaria vaccine antigen, comprising contacting a sample with a monoclonal antibody or antibody fragment that binds to a polypeptide consisting of the amino acid sequence of SEQ ID NO: 1.
23. The method of item 22, wherein the monoclonal antibody or antibody fragment is the monoclonal antibody or antibody fragment of any one of items 1-16, or a combination thereof.
24. The method of item 22 or 23, wherein the monoclonal antibody or antibody fragment is a combination of the monoclonal antibody or antibody fragment of any one of items 1 to 16 and a different monoclonal antibody or antibody fragment that binds to a polypeptide consisting of the amino acid sequence of SEQ ID NO: 1.

### EFFECTS OF THE INVENTION

The present disclosure can provide a monoclonal antibody or antibody fragment that can treat or prevent malaria disease. Also, the present disclosure can provide a method of detecting or quantifying an Ripr-derived malaria vaccine antigen which is useful for quality control during production of a malaria vaccine containing PfRipr5.

### BRIEF DESCRIPTION OF THE DRAWINGS

Fig. 1 shows the inhibitory activity on malaria parasite growth of mouse monoclonal antibodies (26H4 and 29B11) and a control antibody (4B7).
Fig. 2 shows PfRipr5 fragments used for the epitope analysis.
Fig. 3 shows the reactivity of 26H4, 29B11 or an anti-GST antibody with PfRipr5 fragments (EM1 to EM10).
Fig. 4 shows the reactivity of 26H4, 29B11 or an anti-GST antibody with PfRipr5 fragments (EM10, EM11).
Fig. 5 shows the results of determining affinity indexes such as dissociation constant (KD value) by calculating association rate constant (ka value) and dissociation rate constant (kd value).
Fig. 6 shows the results of detection and quantification of a PfRipr5 malaria vaccine antigen by ELISA using 26H4 or 29B11.
Fig. 7 shows the results of Western blot showing the reactivity of 26H4 or 29B11 with a PfRipr5 malaria vaccine antigen. The "-" is the result under non-reducing conditions and the "+" is the result under reducing conditions.

### DETAILED DESCRIPTION

Abbreviations used in description of an amino acid, a (poly)peptide, or a (poly)nucleotide have meanings as defined in IUPAC-IUB Communication on Biological Nomenclature, Eur. J. Biochem., 138:9(1984), or the "Guidelines for the preparation of the description comprising amino acid sequence or nucleotide sequence" of JPO, or as conventionally used in this technical field.

Rh5 interacting protein (also called Ripr) is a protein considered to be expressed in Plasmodium falciparum at the merozoite stage. Ripr can be identified with PlasmoDB gene code: PF3D7_0323400 (http://plasmodb.org/) or NCBI Reference Sequence: XP_001351305 (https://www.ncbi.nlm.nih.gov/). A representative amino acid sequence of Ripr is shown in SEQ ID NO: 17.

PfRipr5 is a fragment of Ripr consisting of the amino acid sequence at positions 720-934 of SEQ ID NO: 17. In the present disclosure, the amino acid sequence at positions 720-934 of SEQ ID NO: 17 is shown as SEQ ID NO: 1.

CDLSCPSNKVCVIENGKQTCKCSERFVLENGVCICANDYKMEDGINCIAKNKC
KRKEYENICTNPNEMCAYNEETDIVKCECKEHYYRSSRGECILNDYCKDINCKE
NEECSIVNFKPECVCKENLKKNNKGECIYENSCLINEGNCPKDSKCIYREYKPHE
CVCNKQGHVAVNGKCVLEDKCVHNKKCSENSICVNVMNKEPICVCTYNYYKK
D (SEQ ID NO: 1)

As used herein, the term "monoclonal antibody" refers to an immunoglobulin molecule comprising two light chains and two heavy chains and includes a chimeric antibody, a humanized antibody, a human antibody, and a multispecific antibody (e.g., bispecific antibody). The term "antibody fragment" refers to a molecule comprising a portion of an immunoglobulin molecule and having an antigen-binding activity, and includes a single-chain antibody (Single-Chain FV, scFV), F(ab')2, Fab', Fab, and Fv.

As used herein, an epitope is a region of an antigen to which an antibody binds. The epitope may comprise a chemically active surface group such as an amino acid, a sugar side chain, or a phosphoryl or sulfonyl group, and may have a characteristic three dimensional structure, and/or a characteristic charge. As used herein, a monoclonal antibody or antibody fragment may be said to specifically bind to an antigen or epitope if it preferentially recognizes that antigen or epitope in a mixture of antigenic substances such as proteins or peptides.

The antibody can be of any class of IgG, IgM, IgA, IgD, and IgE. The basic structure of an antibody molecule is common to each class and composed of heavy chains with molecular weights of 50,000 to 70,000 and light chains with molecular weights of 20,000 to 30,000. A heavy chain is a polypeptide generally containing about 440 amino acids and has a characteristic structure for each class. Heavy chains corresponding to IgG, IgM, IgA, IgD, and IgE are called γ chain, µ chain, α chain, δ chain, and ε chain, respectively. IgG has subclasses of IgG1, IgG2, IgG3, and IgG4, which are called y1, y2, y3, and y4, respectively. A light chain is a polypeptide generally containing about 220 amino acids and known to be any of L and K forms, which are called λ and κ chains, respectively. The basic structure of an antibody molecule includes two heavy chains and two light chains bound by disulfide bonds (SS bonds) and non-covalent bonds, and has a molecular weight of 150,000 to 190,000. The two heavy chains and the two light chains in an antibody molecule may be the same or different from each other.

Heavy chains have 4 (γ, α, and δ chains) or 5 (µ and ε chains) and light chains have 2 intrachain S-S bonds, and one loop every 100-110 amino acids is formed. The conformation of each loop is common and it is called a structural unit or domain. The domain located at the N-terminus of both heavy and light chains has a variable amino acid sequence even in antibodies of the same class (subclass) of allogeneic animals, and it is called a variable region (V region). A heavy chain variable region and a light chain variable region are sometimes referred to as V_{H} and V_{L}, respectively. The amino acid sequence on the C-terminal side from this region is almost constant for each class or subclass, and it is called a constant region (C region). Domains of a heavy chain constant region and a light chain constant region are sometimes referred to as C_{H}1, C_{H}2, and C_{H}3 and C_{L}, respectively.

The antigen-binding portion of an antibody is composed of V_{H} and V_{L}, and the specificity of binding is determined by the amino acid sequences of this portion. On the other hand, biological activities such as binding to complements and various cells depend on the structure of the C region of each immunoglobulin class. The variability of the heavy and light chain variable regions is known to be conferred by three small hypervariable regions present on either chain, and these regions are called complementarity determining regions (CDRs). Several numbering systems are commonly used for determining CDRs, including Kabat, Chothia, AbM, IMGT (Martin et al. (1989) Proc. Natl. Acad. Sci. USA 86: 9268-9272; Martin et al. (1991) Methods Enzymol. 203: 121-153; Pedersen et al. (1992) Immunomethods 1: 126; Reeset al. (1996) In Sternberg M. J.E. (ed.), Protein Structure Prediction, Oxford University Press, Oxford, pp. 141-172 ; Lefranc et al. (2003) Dev Comp Immunol. 27: 55-77). Online tools such as IMGT/V-QUEST (http://www.imgt.org/) have also been used to identify CDRs. In the present disclosure, unless otherwise stated, CDRs of heavy and light chain variable regions are determined by IMGT.

Portions of a variable region other than CDRs are called framework regions (FRs), and their amino acid sequences are relatively constant. The framework regions have a β-sheet structure and CDRs can form loops connecting the β-sheet structures. The CDRs of the heavy and light chains are held in the three-dimensional structure by framework regions and form the antigen-binding portion.

Antigen binding can be confirmed by various assays, such as Surface Plasmon Resonance (SPR), Western blot, direct or indirect sandwich assay, flow cytometry, and immunoprecipitation assay (Zola, Monoclonal Antibodies: A Manual of Techniques, CRC Press, Inc. 1987, pp. 147-158). In certain embodiments, the antigen binding is confirmed by SPR. The SPR is a method of detecting changes in mass on a sensor chip as changes in the angle of disappearance of reflected light caused by surface plasmon resonance and used to analyze the binding specificity and binding affinity between substances, and to measure analyte concentrations in samples.

The present disclosure demonstrates that an antibody that binds to the amino acid sequence at positions 720- 934 of SEQ ID NO: 17, that is, PfRipr5 consisting of the amino acid sequence of SEQ ID NO: 1, has an inhibitory activity on growth of malaria parasites at the red blood cell stage. In certain embodiments, the monoclonal antibody or antibody fragment of the present disclosure binds to the amino acid sequence of SEQ ID NO: 1. In further embodiments, the monoclonal antibody or antibody fragment of the present disclosure binds to an epitope consisting of 5 to 20 contiguous amino acids in the amino acid sequence of SEQ ID NO: 1. In certain embodiments, the epitope consists of an amino acid sequence contained in the amino acid sequence at positions 826-845 of SEQ ID NO: 17, i.e., the amino acid sequence of ENEECSIVNFKPECVCKENL (SEQ ID NO: 2).

Epitope analysis can be performed in various ways (EpitopeMapping Protocols/Second Edition, Mike Schutkowski, Ulrich Reineke, Ann NY AcadSci. 2010 Jan; 1183:267-87.). A more detailed analysis of epitopes can be performed by Western blot using polypeptide fragments.

Binding affinity can be determined by SPR. Devices that measure interactions between substances by SPR generally employ the Kretschmann arrangement using a prism. When polarized light is irradiated so that it is totally reflected on the surface of the metal thin film of the sensor chip, an SPR signal with reduced reflected light intensity is observed in part of the reflected light. The angle at which the SPR signal is generated changes depending on the mass on the sensor chip, and thus association or dissociation between molecules immobilized on the sensor chip (ligands) and molecules added (analytes) changes the angle at which the SPR signal is generated. The measurement results are acquired as sensorgrams showing the angular changes of the SPR signal or the Response Unit (RU) converted from the angular changes over time. By fitting the obtained association and dissociation sensorgrams to the theoretical curve, the association rate constant (ka), dissociation rate constant (kd), and dissociation constant (KD = kd/ka) of the antibody to the antigen can be obtained. In certain embodiments, the SPR is performed under the conditions described in the Examples.

In certain embodiments, the monoclonal antibody or antibody fragment comprises
a heavy chain variable region comprising
   CDR1 consisting of the amino acid sequence of SEQ ID NO: 3 or an amino acid sequence in which one or two amino acid residues are independently substituted, deleted, added, or inserted in the amino acid sequence of SEQ ID NO: 3;
   CDR2 consisting of the amino acid sequence of SEQ ID NO: 4 or SEQ ID NO: 9 or an amino acid sequence in which one or two amino acid residues are independently substituted, deleted, added, or inserted in the amino acid sequence of SEQ ID NO: 4 or SEQ ID NO: 9; and
   CDR3 consisting of the amino acid sequence of SEQ ID NO: 5 or SEQ ID NO: 10 or an amino acid sequence in which one or two amino acid residues are independently substituted, deleted, added, or inserted in the amino acid sequence of SEQ ID NO: 5 or SEQ ID NO: 10; and
a light chain variable region comprising
   CDR1 consisting of the amino acid sequence of SEQ ID NO: 6 or SEQ ID NO: 11 or an amino acid sequence in which one or two amino acid residues are independently substituted, deleted, added, or inserted in the amino acid sequence of SEQ ID NO: 6 or SEQ ID NO: 11;
   CDR2 consisting of the amino acid sequence of SEQ ID NO: 7 or an amino acid sequence in which one or two amino acid residues are independently substituted, deleted, added, or inserted in the amino acid sequence of SEQ ID NO: 7; and
   CDR3 consisting of the amino acid sequence of SEQ ID NO: 8 or SEQ ID NO: 12 or an amino acid sequence in which one or two amino acid residues are independently substituted, deleted, added, or inserted in the amino acid sequence of SEQ ID NO: 8 or SEQ ID NO: 12.

In further embodiments, the monoclonal antibody or antibody fragment comprises
a heavy chain variable region comprising CDR1 consisting of the amino acid sequence of SEQ ID NO: 3, CDR2 consisting of the amino acid sequence of SEQ ID NO: 4 or SEQ ID NO: 9, and CDR3 consisting of the amino acid sequence of SEQ ID NO: 5 or SEQ ID NO: 10; and
a light chain variable region comprising CDR1 consisting of the amino acid sequence of SEQ ID NO: 6 or SEQ ID NO: 11, CDR2 consisting of the amino acid sequence of SEQ ID NO: 7, and CDR3 consisting of the amino acid sequence of SEQ ID NO: 8 or SEQ ID NO: 12.

In further embodiments, the monoclonal antibody or antibody fragment comprises
a heavy chain variable region comprising CDR1 consisting of the amino acid sequence of SEQ ID NO: 3, CDR2 consisting of the amino acid sequence of SEQ ID NO: 4, and CDR3 consisting of the amino acid sequence of SEQ ID NO: 5; and
a light chain variable region comprising CDR1 consisting of the amino acid sequence of SEQ ID NO: 6, CDR2 consisting of the amino acid sequence of SEQ ID NO: 7, and CDR3 consisting of the amino acid sequence of SEQ ID NO: 8.

In further embodiments, the monoclonal antibody or antibody fragment comprises
a heavy chain variable region comprising CDR1 consisting of the amino acid sequence of SEQ ID NO: 3, CDR2 consisting of the amino acid sequence of SEQ ID NO: 9, and CDR3 consisting of the amino acid sequence of SEQ ID NO: 10; and
a light chain variable region comprising CDR1 consisting of the amino acid sequence of SEQ ID NO: 11, CDR2 consisting of the amino acid sequence of SEQ ID NO: 7, and CDR3 consisting of the amino acid sequence of SEQ ID NO: 12.

In further embodiments, the monoclonal antibody or antibody fragment comprises a heavy chain variable region comprising an amino acid sequence having at least 80%, 90%, 95%, 97%, or 99% sequence identity with SEQ ID NO: 13 and a light chain variable region comprising an amino acid sequence having at least 80%, 90%, 95%, 97%, or 99% sequence identity with SEQ ID NO: 14. In further embodiments, the monoclonal antibody or antibody fragment comprises a heavy chain variable region comprising the amino acid sequence of SEQ ID NO: 13 and a light chain variable region comprising the amino acid sequence of SEQ ID NO: 14. In further embodiments, the monoclonal antibody or antibody fragment comprises a heavy chain variable region consisting of the amino acid sequence of SEQ ID NO: 13 and a light chain variable region consisting of the amino acid sequence of SEQ ID NO: 14.

In further embodiments, the monoclonal antibody or antibody fragment comprises a heavy chain variable region comprising an amino acid sequence having at least 80%, 90%, 95%, 97%, or 99% sequence identity with SEQ ID NO: 15 and a light chain variable region comprising an amino acid sequence having at least 80%, 90%, 95%, 97%, or 99% sequence identity with SEQ ID NO: 16. In further embodiments, the monoclonal antibody or antibody fragment comprises a heavy chain variable region comprising the amino acid sequence of SEQ ID NO: 15 and a light chain variable region comprising the amino acid sequence of SEQ ID NO: 16. In further embodiments, the monoclonal antibody or antibody fragment comprises a heavy chain variable region consisting of the amino acid sequence of SEQ ID NO: 15 and a light chain variable region consisting of the amino acid sequence of SEQ ID NO: 16.

As used herein, the term "sequence identity" refers to the proportion of identical amino acid residues between two sequences that are optimally aligned (maximally matched) over the entire region of the sequences to be compared. In the optimal alignment of two sequences, one sequence may have an addition or deletion (eg, gap). The sequence identity can be calculated, for example, by creating an alignment with the Clustal W algorithm (Nucleic Acid Res., 22(22):4673-4680 (1994)). The sequence identity can be calculated by using sequence analysis software such as Vector NTI and GENETYX-MAC, and analysis tools provided in public databases (eg, http://www.ddbj.nig.ac.jp).

In further embodiments, the monoclonal antibody or antibody fragment comprises
a heavy chain variable region comprising CDR1, CDR2 and CDR3 from the amino acid sequence of SEQ ID NO: 13 and a light chain variable region comprising CDR1, CDR2 and CDR3 from the amino acid sequence of SEQ ID NO:14; or
a heavy chain variable region comprising CDR1, CDR2 and CDR3 from the amino acid sequence of SEQ ID NO: 15 and a light chain variable region comprising CDR1, CDR2 and CDR3 from the amino acid sequence of SEQ ID NO:16. In these embodiments, the numbering system for determining CDRs is not limited to any specific system, and may be Kabat, Chothia, AbM, or IMGT, for example.

The monoclonal antibody or antibody fragment of the present disclosure preferably has an inhibitory activity on malaria parasite growth equivalent to or greater than that of any one of the following monoclonal antibodies or antibody fragments of (a) to (d):
(a) a monoclonal antibody or antibody fragment comprising a heavy chain variable region comprising CDR1 consisting of the amino acid sequence of SEQ ID NO: 3, CDR2 consisting of the amino acid sequence of SEQ ID NO: 4, and CDR3 consisting of the amino acid sequence of SEQ ID NO: 5, and a light chain variable region comprising CDR1 consisting of the amino acid sequence of SEQ ID NO: 6, CDR2 consisting of the amino acid sequence of SEQ ID NO: 7, and CDR3 consisting of the amino acid sequence of SEQ ID NO: 8;
(b) a monoclonal antibody or antibody fragment comprising a heavy chain variable region comprising CDR1 consisting of the amino acid sequence of SEQ ID NO: 3, CDR2 consisting of the amino acid sequence of SEQ ID NO: 9, and CDR3 consisting of the amino acid sequence of SEQ ID NO: 10, and a light chain variable region comprising CDR1 consisting of the amino acid sequence of SEQ ID NO: 11, CDR2 consisting of the amino acid sequence of SEQ ID NO: 7, and CDR3 consisting of the amino acid sequence of SEQ ID NO: 12;
(c) a monoclonal antibody or antibody fragment comprising a heavy chain variable region consisting of the amino acid sequence of SEQ ID NO: 13 and a light chain variable region consisting of the amino acid sequence of SEQ ID NO: 14; and
(d) a monoclonal antibody or antibody fragment comprising a heavy chain variable region consisting of the amino acid sequence of SEQ ID NO: 15 and a light chain variable region consisting of the amino acid sequence of SEQ ID NO: 16.

The inhibitory activity on malaria parasite growth can be measured as described in Examples. Specifically, human erythrocytes with a hematocrit of 1% and parasitemia of 0.3-0.6% (parasitemia is the percentage of erythrocytes infected with malaria parasites) which are obtained by mixing non-malaria parasite-infected human erythrocytes and malaria parasite-infected human erythrocytes are cultured with a monoclonal antibody or antibody fragment (0-15 mg/mL) for 25 hours, and the number of erythrocytes infected with malaria parasites is quantified, for example, by staining the nucleus of the parasite with Cyber Green. The inhibitory rate of malaria parasite growth is calculated by obtaining the number of malaria parasite-infected erythrocytes in the treated group considering the number of malaria parasite-infected erythrocytes in the untreated group as 100 and subtracting the number thus obtained from 100, and it is considered a value showing the inhibitory activity on malaria parasite growth. As used herein, the expression that an antibody or antibody fragment has an inhibitory activity on malaria parasite growth equivalent to or greater than that of a given monoclonal antibody or antibody fragment means that the antibody or antibody fragment has ±20% or more of the best inhibitory activity on malaria parasite growth of the given monoclonal antibody or antibody fragment. In certain embodiments, the monoclonal antibody or antibody fragment has 20%, 30%, 40%, or 50% or more inhibitory activity on malaria parasite growth. A monoclonal antibody or antibody fragment may have the inhibitory activity on malaria parasite growth as defined at any concentration tested.

The present disclosure also includes a monoclonal antibody or antibody fragment that competes with any of the monoclonal antibodies or antibody fragments described above. In certain embodiments, the monoclonal antibody or antibody fragment competes with the monoclonal antibody or antibody fragment of any one of (a) to (d) above for binding to a polypeptide consisting of the amino acid sequence of SEQ ID NO: 1. The expression that an antibody or antibody fragment competes with a different antibody or antibody fragment for binding to a polypeptide consisting of the amino acid sequence of SEQ ID NO: 1 means that the antibody or antibody fragment significantly decreases the binding of the different antibody or antibody fragment to the polypeptide consisting of the amino acid sequence of SEQ ID NO: 1. In certain embodiments, the monoclonal antibody or antibody fragment is capable of decreasing the binding of the monoclonal antibody or antibody fragment of any one of (a) to (d) to a polypeptide consisting of the amino acid sequence of SEQ ID NO: 1 by 10%, 20%, 30%, 40%, 50%, 60%, 70%, 80%, 90%, or 95% or more.

The monoclonal antibody or antibody fragment of the present disclosure preferably has a reduced risk of antigenicity when administered to humans. The monoclonal antibody or antibody fragment can be a fully human antibody (also referred to simply as human antibody), a humanized antibody or a chimeric antibody (eg, mousehuman chimeric antibody), or a fragment thereof. A human antibody is an antibody in which all sequences are human sequences, a humanized antibody is an antibody in which sequences other than CDRs are human sequences, and a chimeric antibody is an antibody in which sequences of the variable regions are non-human sequences and those of the constant regions are human sequences. A humanized antibody and chimeric antibody can be prepared by genetic engineering techniques in standard ways. A human antibody can be prepared by using a human-human (or human-mouse) hybridoma, or by phage display or by using a human antibody-producing animal (eg, mouse). An antibody fragment can be prepared by genetic engineering techniques or obtained from a library such as a Fab expression library.

The monoclonal antibody or antibody fragment of the present disclosure may be a multispecific antibody or fragment thereof that binds a polypeptide consisting of the amino acid sequence of SEQ ID NO: 1 and a different antigen.

The monoclonal antibody or antibody fragment may have a modified constant region for regulation of antibody-dependent cellular cytotoxicity (ADCC), complementdependent cellular cytotoxicity (CDC), *in vivo* pharmacokinetics, or other properties. Modifications include modifications of amino acid sequences or sugar chains.

The monoclonal antibody or antibody fragment may be conjugated to a drug. The drug can be a small compound, peptide, or nucleic acid.

The monoclonal antibody or antibody fragment of the present disclosure is useful for the treatment or prevention of malaria disease. That is, the monoclonal antibody or antibody fragment of the present disclosure can be an active ingredient of a pharmaceutical composition for treating or preventing malaria disease. Prevention of malaria disease includes prevention of malaria parasite infection and prevention of development of malaria disease after malaria parasite infection. "Prevention of malaria parasite infection" means prevention of infection of a host with malaria parasites. "Prevention of development of malaria disease after malaria parasite infection" means prevention of manifestation of any of symptoms that will be exhibited by the host due to malaria parasite infection (eg, high fever, headache, nausea, disturbance in consciousness, renal failure). "Treatment of malaria disease" means curing or relieving any of symptoms caused by malaria parasite infection.

A pharmaceutical composition may comprise, in addition to the monoclonal antibody or antibody fragment, a pharmaceutically acceptable carrier or additive such as sterile water, saline, stabilizer, excipient, antioxidant, buffer, preservative, or chelating agent.

The dosage form and administration route of the monoclonal antibody or antibody fragment to a subject can be appropriately determined according to the purpose of administration, condition of the subject or other factors. Examples of dosage forms includes injections, transdermal formulations, inhalants, nasal drops, and oral formulations. In certain embodiments, the dosage form is an injection. Examples of administration routes include intravenous, intraarterial, subcutaneous, intramuscular, transdermal, nasal and oral administrations. In certain embodiments, the administration route is intravenous administration (eg, to an antecubital or any other peripheral vein) or subcutaneous administration.

The dose of the monoclonal antibody or antibody fragment can be appropriately determined according to the purpose of administration, age and body weight of the subject or other factors. The dose can be selected, for example, within the range of 0.0001 mg to 1,000 mg/kg body weight per dose, or can also be selected within the range of 0.001 to 100,000 mg per patient, but is not limited thereto.

The administration schedule of the monoclonal antibody or antibody fragment can be appropriately determined according to the purpose of administration, age and body weight of the subject or other factors. For example, the frequency of administration typically may range from once a week to once every 3 months, more preferably from once every 2 weeks to once every 10 weeks, e.g., once every 4 to 8 weeks. For prevention, typically, the frequency of administration may be once a month to once every 2 to 3 months, or less preferably. The administration may be one time or multiple times (for example, 2 to 5 times) at intervals of several days or weeks.

The monoclonal antibody or antibody fragment of the present disclosure can detect or quantify an Ripr-derived malaria vaccine antigen, particularly PfRipr5, and is useful for quality control of malaria vaccines containing this antigen or intermediate products during production of the vaccines. Quality control includes standard control and process control. In certain aspects, the present disclosure provides a method of detecting or quantifying an Ripr-derived malaria vaccine antigen comprising contacting the monoclonal antibody or antibody fragment of the present disclosure with a sample. The monoclonal antibody or antibody fragment to be used may be one or more, and can be a monoclonal antibody or antibody fragment that binds to an epitope consisting of 5 to 20 consecutive amino acids in the amino acid sequence of SEQ ID NO: 1, a different monoclonal antibody or antibody fragment that binds to a polypeptide consisting of the amino acid sequence of SEQ ID NO: 1, or a combination thereof. In certain embodiments, the Ripr-derived malaria vaccine antigen comprises or consists of an amino acid sequence having at least 80%, 90%, 95%, 97%, or 99% sequence identity with the amino acid sequence of SEQ ID NO: 1. In further embodiments, the Ripr-derived malaria vaccine antigen comprises or consists of the amino acid sequence of SEQ ID NO: 1. The sample can be, for example, a malaria vaccine containing an Ripr-derived malaria vaccine antigen or a sample obtained in the production process of the vaccine. Detection or quantification can be performed, for example, by ELISA or Western blot.

### EXAMPLES

The present invention will be described in detail below with reference to Examples, but the present invention is not limited thereto.

### 1. Production of mouse monoclonal antibody

Two Balb/c mice were immunized by hock immunization a total of 3 times with Ripr along with Titer-MAX Gold Adjuvant^{®}. Cell fusion of the spleen- and lymph nodederived lymphocytes of the mice with mouse myeloma cell P3U1 was performed. The culture supernatant of the obtained cells was screened by ELISA for the binding to Ripr. A sample showing a value 100 times or more that of normal serum before immunization was defined as positive, and the cells of the positive sample were cultured in a 24-well plate. The culture supernatant of the cells was screened by immunofluorescence staining for the binding to malaria parasites. For clones that showed binding to malaria parasites, cloning by limiting dilution were carried out twice to obtain antibody-producing hybridomas. The established hybridomas were further expanded to determine the antibody subclass of the mouse monoclonal antibody produced by each clone.

A plurality of antibodies having binding activity to PfRipr5 (SEQ ID NO: 1) were obtained. Among them, 11 clones (8A6, 10B10, 10G5, 11D5, 24D2, 26F10, 26H4, 27F11, 29B11, 29H7, 30G12) were determined to be positive for binding to malaria parasites by immunofluorescence staining.

### 2. Evaluation of inhibitory activity on malaria parasite growth

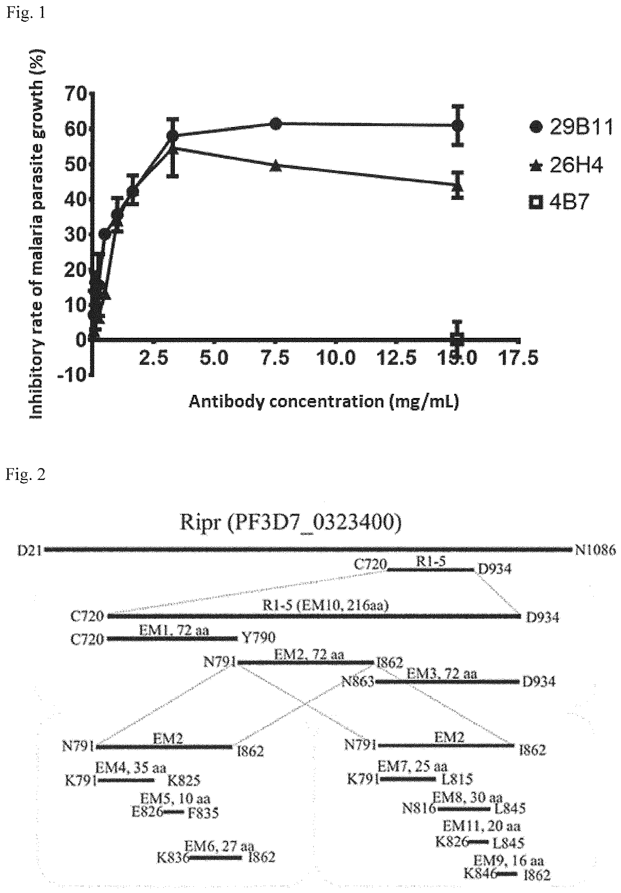

Human erythrocytes with a hematocrit of 1% and parasitemia of 0.3-0.6% which were obtained by mixing non-malaria parasite-infected human erythrocytes and malaria parasite-infected human erythrocytes were cultured with a mouse monoclonal antibody (0.029-15 mg/mL) for 25 hours. 4B7, a monoclonal antibody that specifically binds to the surface protein Pfs25 of Plasmodium falciparum zygotes and ookinates and has a propagation-blocking effect, was used as a control antibody. After the culturing, the nucleus of the parasite was stained with Cyber Green, and the number of erythrocytes infected with malaria parasites was quantified by FACS. The inhibitory rate of malaria parasite growth was calculated by obtaining the number of malaria parasite-infected erythrocytes in each antibody-treated group considering the number of malaria parasite-infected erythrocytes in the antibody-untreated group as 100, and subtracting the number thus obtained from 100. As shown in Fig. 1, among the 11 clones obtained in section 1 above, monoclonal antibodies 26H4 and 29B11 exhibited a strong inhibitory activity on malaria parasite growth.

### 3. Preparation of polypeptides

A vector comprising a wheat codon-optimized synthetic DNA encoding PfRipr5 attached to a His-tag coding sequence at the C-terminus, named pEU-E01-MCS, or a vector comprising a sequence of one of 10 fragments (EM1 to EM10, Fig. 2) subcloned from the codon-optimized DNA attached to a His-tag coding sequence at the C-terminus, named pEU-E01-GST-TEV-N2, was used as the template for transcription. The template for synthesizing each polypeptide comprised a Met coding sequence at the N-terminus. The whole transcribed mRNA was used for protein synthesis with a wheat germ cell-free protein synthesis kit WEPRO^{®} 7240H (cell free Science). The resulting solution containing the synthesized protein was affinity purified.

### 4. Western blot analysis

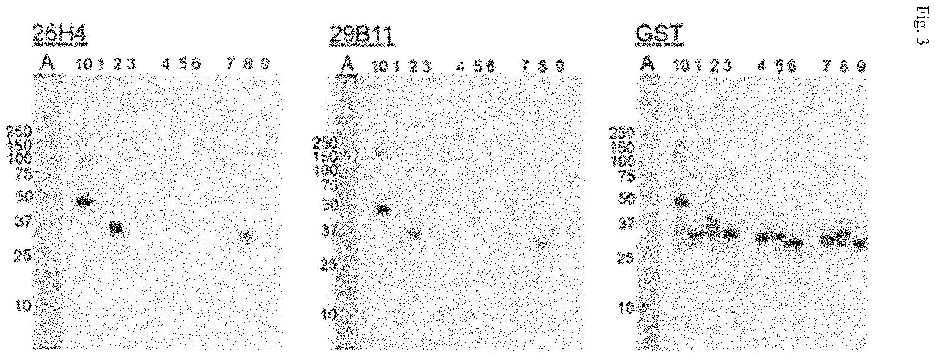

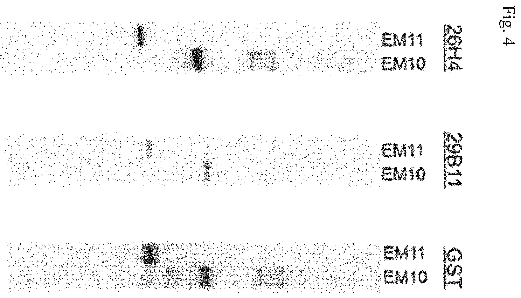

The prepared polypeptide samples were incubated at 37°C for 30 minutes and centrifuged at 10,000 g at 4°C for 10 minutes, and its supernatant was collected and separated by SDS-PAGE on Bolt^{™} 4-12% Bis-Tris Plus (Invitrogen). After electroblotting onto a membrane (Hybond LFP; GE Healthcare) using a semi-dry blotting system (Bio-Rad), the membrane was incubated with a diluted mouse monoclonal antibody (26H4 or 29B11) (1:100 dilution) or a rabbit anti-GST antibody (1:1000 dilution) (Santa Cruz Biotechnology) and then washed with buffer. The membrane was probed with an enzyme-labeled secondary antibody (GE Healthcare) and visualized by using a chemiluminescent substrate (Millipore) and LAS 4000 image analyzer (GE Healthcare). Molecular weights of proteins were estimated by using Precision Plus (Bio-Rad). As shown in Fig. 3, EM2 (72 amino acids) and EM8 (30 amino acids) fragments reacted with 26H4 and 29B11 in the Western blot. When shorter fragments were examined for reaction, as shown in Fig. 4, EM11 fragment (20 amino acids at positions 826-845 in the full length Ripr; SEQ ID NO: 2) reacted with 26H4 and 29B11 in the Western blot. These results suggested that the amino acid sequence of SEQ ID NO: 2 contained an epitope.

### 5. SPR analysis

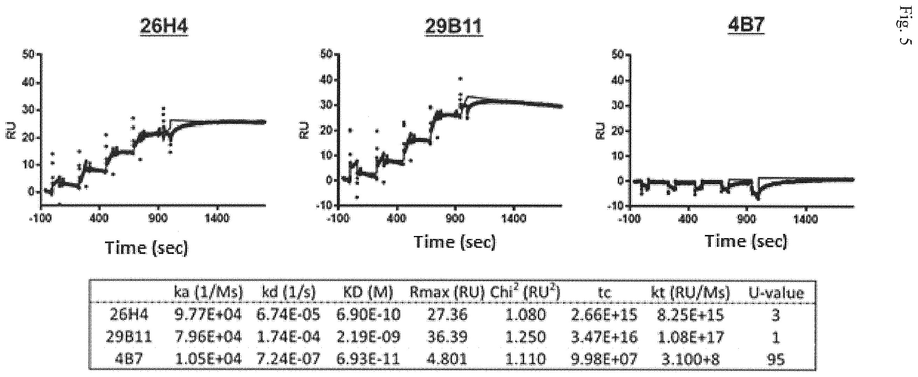

SPR analysis was performed according to the instruction manual of the Biacore X100 instrument (GE Healthcare). Binding affinity analysis was performed using the BiacoreX100 evaluation software. Sensor chip CM5, amine coupling reagents, Mouse antibody capture kit, and buffers were purchased from GE Healthcare. As the running buffer, 10 mM HEPES, pH 7.4, 150 mM NaCl, 3 mM EDTA, 0.05% (v/v) P20 was used. The anti-mouse antibody immobilized on the Sensor chip CM5 was allowed to capture a mouse monoclonal antibody to be tested and used as a ligand. All assays were performed at antigen concentrations of 18.75, 37.5, 75, 150, and 300 nM with a contact time of 120 seconds and a dissociation time of 180 seconds. After subtraction of buffer effects and non-specific interactions with the surface of the Sensor chip CM5 using blank flow cells, global fitting of the data yielded the association rate constant (ka) and dissociation rate constant (kd). Fig. 5 shows the analysis results.

### 6. CDR analysis

Total RNA was prepared from hybridoma cell pellets. After quality testing by Agilent bioanalyzer analysis, reverse transcription reaction was performed by using the total RNA as the template with SMARTer RACE 5'/3' Kit (TaKaRa Code Z4859N) and a mouse antibody (IgG) heavy chain constant region-specific primer. By using the resulting cDNA as the template, RACE PCR was performed with a heavy chain constant region-specific primer. The resulting PCR products were purified and cloned into plasmid vectors, and then nucleotide sequences of 48 randomly picked clones were analyzed. By using the total RNA as the template, the light chain was also analyzed in the same way. The analysis results were assembled to obtain a consensus sequence, and the encoded amino acid sequence was deduced. Also, heavy and light chain CDRs were determined by IMGT/V-QUEST (http://www.imgt.org/).

The amino acid sequences of heavy chain CDR1-3 (H-CDR1-3), light chain CDR1-3 (L-CDR1-3), heavy chain variable region (V_{H}), and light chain variable region (V_{L}) of the mouse monoclonal antibodies 26H4 and 29B11 are shown in Table 1.

**Table 1**

| Antibody | | Amino Acid Sequence | SEQ ID NO: |
|---|---|---|---|
| 26114 | H-CDR1 | GYTFTEYT | 3 |
| | H-CDR2 | IDPNNGVT | 4 |
| | H-CDR3 | ARYYYGIIYFDY | 5 |
| | L-CDR1 | IVHSNGNTY | 6 |
| | L-CDR2 | KVS | 7 |
| | L-CDR3 | FQNSHVTWT | 8 |
| | V_{H} | GNNICPMSSPQTLNTLTLTMGWSWIFLFLLSGTAGVLSEVQLQQSG PELVKPGASVKISCKTSGYTFTEYTMHWVKQSHGKSLEWIGGIDPN NGVTIYNQNFKGKATLTVDKSSSTAYMELRSLTSEDSAVYHCARYY YGHYFDYWGQGTTLTVSSAKTTPPSVYPLAPGSAAQTNSMVTLGCL VKGYF | 13 |
| | V_{L} | AVVNAVYMGTDQSPQAVSSGCLLKMKLPVRLLVLMFWIPGSSSDVL MTQTPLSLPVSLGDQASISCRSSQSIVHSNGNTYLEWYLQKPGQSP KLLIYKVSNRFSGVPDRFSGSGSGTDFTLKISRVEAEDLGVYYCFQ NSHVTWTFGGGTKLEIKRADAAPTVSIFPPSSEQLTSGGASVVCFL NNFYPKDINVKWKIDGS | 14 |
| 29811 | H-CDR1 | GYTFTEYT | 3 |
| | H-CDR2 | IDPNNGIT | 9 |
| | H-CDR3 | TRYYYGHYFDY | 10 |
| | L-CDR1 | IVHNNGHTY | 11 |
| | L-CDR2 | KVS | 7 |
| | L-CDR3 | FQDSHVPWT | 12 |
| | V_{H} | DPENNICPMSSPQTLNTLTLTMGWSWIFLFLLSGTAGVLSEVQLQQ SGPELMKPGASVKISCKTSGYTFTEYTMHWVKQSHGKSLEWIGGID PNNGITIYDQKFKGKATLTVDKSSSTAYMELRSLTSEDSAVYYCTR YYYGHYFDYWGQGTTLTVSSAKTTPPSVYPLAPGSAAQTNSMVTLG CLVKGYF | 15 |
| | V_{L} | VSVTDQSPQAVSSGCLLKMKLPVRLLVLMFWIPASSSDVLMTQTPL SLPVSLGDQASISCRSSQSIVHNNGHTYLEWYLQKPGQSPKLLIYK VSNRFSGVPDRFSGSGSGTDFTLKISRVEAEDLGIYYCFQDSHVPW TFGGGTKLEIKRADAAPTVSIFPPSSEQLTSGGASVVCFLNNFYPK DINVKWKIDGS | 16 |

### 7. Malaria Vaccine Quality Control (ELISA)

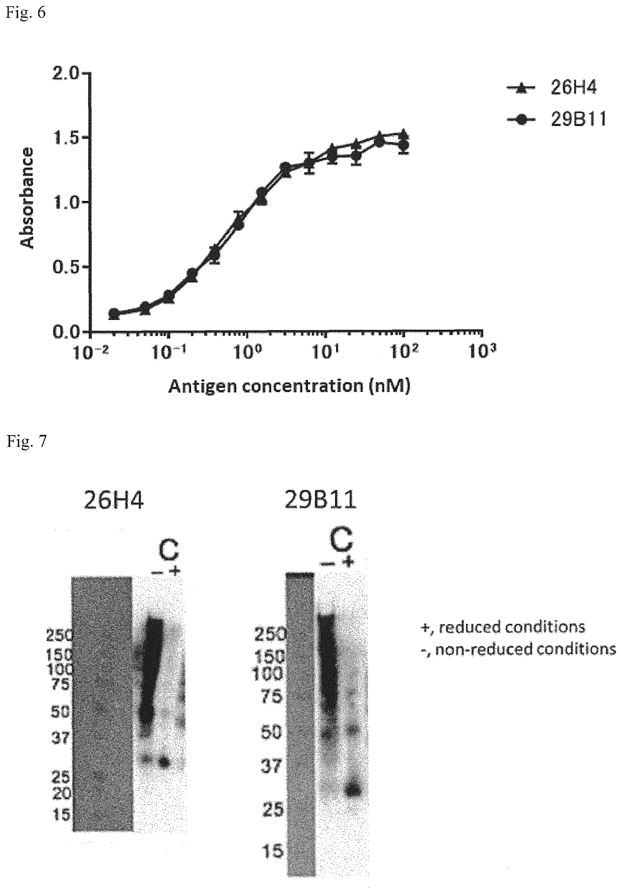

The PfRipr5 malaria vaccine antigen contained in the sample prepared by the wheat germ cell-free protein synthesis technique as described in section 3 above was detected and quantified by ELISA as follows. The mouse monoclonal antibody 26H4 or 29B11 (2.5 µg/ml) diluted in carbonate buffer was attached to a 96-well plate and a diluted solution containing the test sample in phosphate buffer (100 nM solution was serially diluted at a common ratio of 2; 0.02 nM to 100 nM) was added to the plate, and then the plate was blocked with phosphate buffer containing 0.05% Tween 20 and 3% skim milk. An anti-Ripr polyclonal antibody prepared from rabbit antiserum was diluted 2000-fold with phosphate buffer and added to the blocked plate, and the plate was then incubated. Further, a secondary antibody (Anti-Rabbit IgG, HRP-Linked Whole Ab Donkey, GE Healthcare) diluted 1000-fold with phosphate buffer was added. A substrate solution (TMB Substrate kit, Thermo Scientific) was then added, and the resulting product of the enzymatic reaction was measured with a microplate reader. As shown in Fig. 6, it was demonstrated that the monoclonal antibody could detect and quantify the PfRipr5 malaria vaccine antigen.

### 8. Malaria Vaccine Quality Control (Western Blot)

The PfRipr5 malaria vaccine antigen contained in the sample prepared by the wheat germ cell-free protein synthesis technique as described in section 3 above was detected by Western blot as follows. The test sample was incubated at 37°C for 30 minutes in the presence or absence of a reducing agent (2-mercaptoethanol) and centrifuged at 10,000 g at 4°C for 10 minutes, and the supernatant was collected and separated by SDS-PAGE. After electroblotting onto a membrane (Hybond LFP; GE Healthcare), the membrane was incubated with the mouse monoclonal antibody 26H4 or 29B11 (1:100 dilution) and then washed with buffer. The membrane was probed with an enzyme-labeled secondary antibody (GE Healthcare) and visualized by using a chemiluminescent substrate (Millipore) and LAS 4000 image analyzer (GE Healthcare). As shown in Fig. 7, the malaria vaccine antigen reacted with the mouse monoclonal antibodies 26H4 and 29B11 in the Western blot, indicating that the monoclonal antibody can be used to detect the PfRipr5 malaria vaccine antigen.

### INDUSTRIAL APPLICABILITY

The antibody or antibody fragment of the present disclosure is useful for treating or preventing malaria disease and for quality control during malaria vaccine production.

### SEQUENCE LISTING FREE TEXT

SEQ ID NO: 1: amino acid sequence at positions 720 -934 of SEQ ID NO: 17
SEQ ID NO: 2: amino acid sequence at positions 826-845 of SEQ ID NO: 17
SEQ ID NO: 3: H-CDR1
SEQ ID NO: 4: H-CDR2
SEQ ID NO: 5: H-CDR3
SEQ ID NO: 6: L-CDR1
SEQ ID NO: 8: L-CDR3
SEQ ID NO: 9: H-CDR2
SEQ ID NO: 10: H-CDR3
SEQ ID NO: 11: L-CDR1
SEQ ID NO: 12: L-CDR3
SEQ ID NO: 13: Heavy chain variable region
SEQ ID NO: 14: Light chain variable region
SEQ ID NO: 15: Heavy chain variable region
SEQ ID NO: 16: Light chain variable region

## Claims

1. A monoclonal antibody or antibody fragment that binds to an epitope consisting of 5 to 20 consecutive amino acids in the amino acid sequence of SEQ ID NO: 1.

2. The monoclonal antibody or antibody fragment of claim 1, wherein the epitope consists of a sequence contained in the amino acid sequence of SEQ ID NO: 2.

3. The monoclonal antibody or antibody fragment of claim 1 or 2, comprising
a heavy chain variable region comprising
CDR1 consisting of the amino acid sequence of SEQ ID NO: 3 or an amino acid sequence in which one or two amino acid residues are independently substituted, deleted, added, or inserted in the amino acid sequence of SEQ ID NO: 3;
CDR2 consisting of the amino acid sequence of SEQ ID NO: 4 or SEQ ID NO: 9 or an amino acid sequence in which one or two amino acid residues are independently substituted, deleted, added, or inserted in the amino acid sequence of SEQ ID NO: 4 or SEQ ID NO: 9; and
CDR3 consisting of the amino acid sequence of SEQ ID NO: 5 or SEQ ID NO: 10 or an amino acid sequence in which one or two amino acid residues are independently substituted, deleted, added, or inserted in the amino acid sequence of SEQ ID NO: 5 or SEQ ID NO: 10; and
a light chain variable region comprising
CDR1 consisting of the amino acid sequence of SEQ ID NO: 6 or SEQ ID NO: 11 or an amino acid sequence in which one or two amino acid residues are independently substituted, deleted, added, or inserted in the amino acid sequence of SEQ ID NO: 6 or SEQ ID NO: 11;
CDR2 consisting of the amino acid sequence of SEQ ID NO: 7 or an amino acid sequence in which one or two amino acid residues are independently substituted, deleted, added, or inserted in the amino acid sequence of SEQ ID NO: 7; and
CDR3 consisting of the amino acid sequence of SEQ ID NO: 8 or SEQ ID NO: 12 or an amino acid sequence in which one or two amino acid residues are independently substituted, deleted, added, or inserted in the amino acid sequence of SEQ ID NO: 8 or SEQ ID NO: 12.

4. The monoclonal antibody or antibody fragment of any one of claims 1-3, comprising
a heavy chain variable region comprising
CDR1 consisting of the amino acid sequence of SEQ ID NO: 3;
CDR2 consisting of the amino acid sequence of SEQ ID NO: 4 or SEQ ID NO: 9; and CDR3 consisting of the amino acid sequence of SEQ ID NO: 5 or SEQ ID NO: 10; and
a light chain variable region comprising
CDR1 consisting of the amino acid sequence of SEQ ID NO: 6 or SEQ ID NO: 11;
CDR2 consisting of the amino acid sequence of SEQ ID NO: 7; and
CDR3 consisting of the amino acid sequence of SEQ ID NO: 8 or SEQ ID NO: 12.

5. The monoclonal antibody or antibody fragment of any one of claims 1-4, comprising
a heavy chain variable region comprising CDR1 consisting of the amino acid sequence of SEQ ID NO: 3, CDR2 consisting of the amino acid sequence of SEQ ID NO: 4, and CDR3 consisting of the amino acid sequence of SEQ ID NO: 5; and
a light chain variable region comprising CDR1 consisting of the amino acid sequence of SEQ ID NO: 6, CDR2 consisting of the amino acid sequence of SEQ ID NO: 7, and CDR3 consisting of the amino acid sequence of SEQ ID NO: 8.

6. The monoclonal antibody or antibody fragment of any one of claims 1-4, comprising
a heavy chain variable region comprising CDR1 consisting of the amino acid sequence of SEQ ID NO: 3, CDR2 consisting of the amino acid sequence of SEQ ID NO: 9, and CDR3 consisting of the amino acid sequence of SEQ ID NO: 10; and
a light chain variable region comprising CDR1 consisting of the amino acid sequence of SEQ ID NO: 11, CDR2 consisting of the amino acid sequence of SEQ ID NO: 7, and CDR3 consisting of the amino acid sequence of SEQ ID NO: 12.

7. The monoclonal antibody or antibody fragment of any one of claims 1-6, comprising a heavy chain variable region comprising an amino acid sequence having at least 90% sequence identity with SEQ ID NO: 13 and a light chain variable region comprising an amino acid sequence having at least 90% sequence identity with SEQ ID NO: 14.

8. The monoclonal antibody or antibody fragment of claim 7, comprising a heavy chain variable region comprising the amino acid sequence of SEQ ID NO: 13 and a light chain variable region comprising the amino acid sequence of SEQ ID NO: 14.

9. The monoclonal antibody or antibody fragment of any one of claims 1-6, comprising a heavy chain variable region comprising an amino acid sequence having at least 90% sequence identity with SEQ ID NO: 15 and a light chain variable region comprising an amino acid sequence having at least 90% sequence identity with SEQ ID NO: 16.

10. The monoclonal antibody or antibody fragment of claim 9, comprising a heavy chain variable region comprising the amino acid sequence of SEQ ID NO: 15 and a light chain variable region comprising the amino acid sequence of SEQ ID NO: 16.

11. The monoclonal antibody or antibody fragment of any one of claims 1-10, comprising
a heavy chain variable region comprising CDR1, CDR2 and CDR3 from the amino acid sequence of SEQ ID NO: 13 and a light chain variable region comprising CDR1, CDR2 and CDR3 from the amino acid sequence of SEQ ID NO: 14; or
a heavy chain variable region comprising CDR1, CDR2 and CDR3 from the amino acid sequence of SEQ ID NO: 15 and a light chain variable region comprising CDR1, CDR2 and CDR3 from the amino acid sequence of SEQ ID NO: 16.

12. A monoclonal antibody or antibody fragment that competes with the monoclonal antibody or antibody fragment of any one of claims 1 to 11 for binding to a polypeptide consisting of the amino acid sequence of SEQ ID NO: 1.

13. The monoclonal antibody or antibody fragment of any one of claims 1 to 12, which is a chimeric antibody or fragment thereof.

14. The monoclonal antibody or antibody fragment of any one of claims 1 to 12, which is a humanized antibody or fragment thereof.

15. The monoclonal antibody or antibody fragment of any one of claims 1-14, which has a modified constant region.

16. The monoclonal antibody or antibody fragment of any one of claims 1-15, which is conjugated to a drug.

17. A pharmaceutical composition for treating or preventing malaria disease, comprising the monoclonal antibody or antibody fragment of any one of claims 1 to 16.

18. A method of detecting or quantifying an Ripr-derived malaria vaccine antigen, comprising contacting a sample with the monoclonal antibody or antibody fragment of any one of claims 1 to 16.
